(19) Europäisches Patentamt
European Patent Office
Office européen des brevets

(11) **EP 4 428 250 A1**

(12) **EUROPEAN PATENT APPLICATION**
published in accordance with Art. 153(4) EPC

(43) Date of publication:
**11.09.2024  Bulletin 2024/37**

(21) Application number: **22890453.8**

(22) Date of filing: **04.11.2022**

(51) International Patent Classification (IPC):
***C12Q 1/6886*** (2018.01)

(52) Cooperative Patent Classification (CPC):
**C12Q 1/6886**

(86) International application number:
**PCT/KR2022/017269**

(87) International publication number:
**WO 2023/080722 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **05.11.2021  KR 20210151412**

(71) Applicants:
• **Seoul National University Hospital**
  **Seoul 03080 (KR)**
• **Seoul National University R & DB Foundation**
  **Seoul 08826 (KR)**

(72) Inventors:
• **KIM, Sun**
  **Seoul 08826 (KR)**
• **LEE, Do Hoon**
  **Seoul 08826 (KR)**
• **KOO, Bon Il**
  **Seoul 08826 (KR)**
• **YOON, Sung-Soo**
  **Seoul 08826 (KR)**
• **KOH, Young Il**
  **Seoul 03080 (KR)**

(74) Representative: **Schwarz & Partner Patentanwälte GmbH**
**Patentanwälte**
**Wipplingerstraße 30**
**1010 Wien (AT)**

(54) **METHOD FOR PREDICTING REACTIVITY TO ANTICANCER DRUGS BY ANALYZING GENOMIC DNA METHYLATION STATUS**

(57)    The present invention relates to a method for predicting reactivity to anticancer drugs by analyzing genomic DNA methylation status and, more specifically to a method for predicting reactivity to anticancer drugs by analyzing the methylation status of adjacent CpG sites in genomic DNA. The method of the present invention can accurately predict reactivity to anticancer drugs by using the methylation disorderness of CpG sites in genomic DNA, and thus can be very useful in the selection of drugs for cancer patient treatment.

FIG. 2

**Description**

**TECHNICAL FIELD**

The present application claims priority to Korean Patent Application No. 2021-0151412 filed on November 5, 2021, and the entire specification thereof is incorporated herein by reference.

The present invention relates to a method for predicting reactivity to anticancer drugs by analyzing genomic DNA methylation status and, more specifically to a method for predicting reactivity to anticancer drugs by analyzing the methylation status of adjacent CpG sites in genomic DNA..

**BACKGROUND ART**

World Health Organization (WHO) reports that the number of new cancer patients is rapidly increasing, and the death rate due to cancer is also increasing every year. Although numerous anticancer drugs have been developed to date for various cancers, only a small number of cancers can be cured with anticancer drugs alone. This is because, when treating cancer using an anticancer drug, the reactivity of a single type of cancer to the anticancer drug may vary depending on individual genetic differences, epigenetic diversity, and environmental influences.

Among them, DNA methylation, which is the most studied epigenetic mutation, mainly occurs at the cytosine of the CpG island in the promoter region of a specific gene such that the binding of transcription factors is interrupted, resulting in gene silencing of the expression of specific genes. This is the main mechanism by which the function of a gene is lost without a mutation in the coding sequence.

It is known that DNA methylation in untranslated regions such as enhancers and regulatory regions, in addition to the promoter region of a gene, also acts together with chromosomal structural variation and histone modification, and is a causative mechanism for various diseases. Such abnormal methylation/demethylation of CpG sites has been reported in various diseases such as cancer, and attempts are being made to investigate promoter methylation of disease-related genes and use same in the diagnosis of various diseases, and to predict reactivity to anticancer drugs by analyzing the methylation status of specific genes.

Although most of the methods being conventionally attempted use the methylation level at CpG islands within a specific gene as a predictive marker for reactivity to anticancer drugs, a method for predicting drug reactivity through changes in the methylation status of CpG across genomic DNA has not been reported yet.

Since the status of genomic DNA methylation is very dynamically regulated, the aspects may vary depending on the type of a cell or its developmental period such that the expression of a set of genes appropriate for each function of a cell is induced.

The low precision of conservation of genomic base sequencing reads to the accumulation of inevitable errors in DNA methylation status during cell division. Therefore, as a result of observing the DNA methylation status of a random region of the genome in a group of cells formed through division, the cells do not have the same status and show a certain level of variability. This is called the disorder of DNA methylation.

Since excessive DNA methylation disorder makes it impossible to maintain tissue-level function, cells maintain the disorder of DNA methylation at an appropriate level through several mechanisms. One example of them is the processivity of DNA methyltransferase itself. DNA methyltransferase is known to move along DNA strands and perform an enzyme action. In other words, if an enzyme transfers a methyl group to a certain CpG site, it tends to move along the DNA strands to another locally adjacent CpG site to perform the enzyme action. Therefore, CpG sites that exist nearby are likely to have the same DNA methylation status, which has the effect of lowering the disorder of DNA methylation in local genomic regions.

Since epigenetic factors such as DNA methylation determine the status of cells by regulating gene expression, it can be said that the diversity of DNA methylation status at the cell group level is an indicator of the diversity of cell status within a cell group. In particular, methylation disorder in cancer can be considered a measure of intratumoral heterogeneity from an epigenetic perspective, and an increase in such value is known to be associated with a poor prognosis for patients in carcinomas such as chronic lymphocytic leukemia. In addition, it has been reported that the diversity of gene expression in cancer cells constituting cancer with increased disorder increases. In this way, it is accepted that methylation disorder increases the heterogeneity of epigenetic tumors and increases the adaptive potential of cancer, thereby facilitating the emergence of more aggressive cancer cells and increasing the risk of cancer.

**SUMMARY OF INVENTION**

**TECHNICAL PROBLEM**

In the present invention, the present inventor has conducted extensive research to develop a method for predicting reactivity to anticancer drugs by evaluating the methylation status of adjacent CpG sites in one or more regions of the genomic DNA of cancer patients, and, as a result, it was discovered that, regardless of the methylation level of the CpG sites, the methylation disorder of adjacent CpG sites in one or more regions of genomic DNA might be an important indicator for predicting reactivity to anticancer drugs, and thus the present invention has been completed.

Accordingly, the object of the present invention is to provide a method for evaluating the methylation status of adjacent CpG sites in genomic DNA in order to provide information necessary for predicting reactivity to anticancer drugs in cancer patients, the method comprising: (a) isolating genomic DNA from a biological sample isolated from a subject; (b) measuring the methylation status of CpG sites in the isolated genomic DNA; and (c) assessing the methylation disorder of adjacent CpG sites in one or more regions in the genomic DNA.

**SOLUTION TO PROBLEM**

In order to achieve the above-described objects of the present invention, the present invention provides a method for evaluating the methylation status of adjacent CpG sites in genomic DNA in order to provide information necessary for predicting reactivity to anticancer drugs in cancer patients, the method comprising: (a) isolating genomic DNA from a biological sample isolated from a subject; (b) measuring the methylation status of CpG sites in the isolated genomic DNA; and (c) assessing the methylation disorder of adjacent CpG sites in one or more regions in the genomic DNA.

Hereinafter, the present invention will be described in detail.

The present invention provides a method for evaluating the methylation status of adjacent CpG sites in genomic DNA in order to provide information necessary for predicting reactivity to anticancer drugs in cancer patients, the method comprising: (a) isolating genomic DNA from a biological sample isolated from a subject; (b) measuring the methylation status of CpG sites in the isolated genomic DNA; and (c) assessing the methylation disorder of adjacent CpG sites in one or more regions in the genomic DNA.

According to one embodiment of the present invention, it has been confirmed that the methylation disorder of adjacent CpG sites in one or more regions within the genomic DNA of blood cancer cells may be an important indicator for predicting reactivity to anticancer drugs. This methylation disorder of CpG sites is independent of the methylation level of the CpG sites, and is based on the assumption that adjacent CpG sites within genomic DNA will show the same methylation status (i.e., methylation or demethylation).

As used herein, the term "CpG" refers to a dinucleotide sequence, in which cytosine nucleotides occur next to guanine nucleotides in a linear sequence of bases along its length. In the CpG sequence, a cytosine nucleotide is 5' to a guanine nucleotide, and the two nucleotides are linked by a phosphate molecule. Cytosines in CpG dinucleotides may be methylated to form 5-methylcytosine. The methylation of cytosines within a gene or promoter may affect the transcriptional regulation of the gene in mammals. The enzyme that adds methyl groups is called DNA methyltransferase.

As used herein, the term "methylation disorder" may be used with the same meaning as "discordant methylation" and refers to the degree of discordance in the methylation status of CpG sites that exists over a short genetic distance or within a specific region of genomic DNA. In general, since DNA methylation changes according to specific regions of genomic DNA (e.g., specific gene promoters or CpG islands) rather than individual CpGs, the degree to which the methylation statuses of CpGs located in close genetic proximity are concordant is expected to be very high under a normal status. For example, it is very likely that CpG sites located adjacent to each other by a genetic distance of 2 to 100 bp are all methylated or all demethylated under a normal status. In a more strict sense, the term may refer to whether methylations of CpG sites contained in the same sequencing read are concordant. If the CpGs contained in a sequencing read are either all methylated or not all methylated (i.e., all demethylated), the sequencing read is classified as concordant methylation. In other cases, it is classified as discordant methylation. In general, when analyzing the sequence of genomic DNA, a sequencing read may have a genetic distance of around 100 base pairs (bp).

As used herein, the term "methylation" refers to the addition of a methyl group to the 5' carbon of the cytosine base in the deoxyribonucleic acid sequence of the CpG site in genomic DNA.

As used herein, the term "methylation status" refers to the presence or absence of a methylated cytosine base at a CpG site.

As used herein, the term "adjacent CpG sites" refers to (i) a set of CpG sites within a specific region of genomic DNA, (ii) a set of CpG sites within a sequencing read, or (iii) a set of CpG sites with a short genetic distance.

As used herein, the term "specific region of genomic DNA" may be selected from a group consisting of an entire genomic DNA, a bivalent domain, a promoter, an enhancer, an exon, an intron, a 5'-untranslated region (UTR), 3'-UTR, a gene body, a stem cell-related region, a CpG island, a CpG shore, a long terminal repeat (LTR), a long interspersed nuclear element (LINE), a short interspersed nuclear element (SINE), a CpG shelf, a methylation canyon and an intergenic region.

As used herein, the terms "short genetic distance," "genetically short distance," "with a short genetic distance," and "in close genetic proximity" may be used with the same meaning and refer to a genetic distance of 2 to 100 bp, specifically, 2 bp, 3 bp, 4 bp, 5 bp, 6 bp, 7 bp, 8 bp, 9 bp, 10 bp, 11 bp, 12 bp, 13 bp, 14 bp, 15 bp, 16 bp, 17 bp, 18 bp, 19 bp, 20 bp, 21 bp, 22 bp, 23 bp, 24 bp, 25 bp, 26 bp, 27 bp, 28 bp, 29 bp, 30 bp, 31 bp, 32 bp, 33 bp, 34 bp, 35 bp, 36 bp, 37 bp, 38 bp, 39 bp, 40 bp, 41 bp, 42 bp, 43 bp, 44 bp, 45 bp, 46 bp, 47 bp, 48 bp, 49 bp, 50 bp, 51 bp, 52 bp, 53 bp, 54 bp, 55 bp, 56 bp, 57 bp, 58 bp, 59 bp, 60 bp, 61 bp, 62 bp, 63 bp, 64 bp, 65 bp, 66 bp, 67 bp, 68 bp, 69 bp, 70 bp, 71 bp, 72 bp, 73 bp, 74 bp, 75 bp, 76 bp, 77 bp, 78 bp, 79 bp, 80 bp, 81 bp, 82 bp, 83 bp, 84 bp, 85 bp, 86 bp, 87 bp, 88 bp, 89 bp, 90 bp, 91 bp, 92 bp, 93 bp, 94 bp, 95 bp, 96 bp, 97 bp, 98 bp, 99 bp or 100 bp. In this respect, the term "adjacent CpG sites" may refer to (i) a set of CpG sites located at short genetic distances within a specific region of genomic DNA, or (ii) a set of CpG sites located at short genetic distances within a sequencing read.

As used herein, the term "drug reactivity" refers to the degree of therapeutic effectiveness of a specific drug on a subject, preferably a cancer patient. For example, when a drug is used in connection with the treatment of cancer patients, the term "increased reactivity," "enhanced reactivity," "good reactivity" or "high reactivity" may refer to an increase in the effectiveness of a drug as measured using any method known in the art. As another example, a cancer patient's reactivity to a drug may be characterized as a complete or partial reaction. As another example, a cancer patient's increased drug reactivity may be characterized by an overall survival, disease-free survival, a desired reaction speed, time to tumor progression, progression-free survival, or time to treatment failure. The object of comparison as a standard for "increased reactivity," "enhanced reactivity," "good reactivity" or "high reactivity" may be the reactivity of the patient who administered the drug, or the average reactivity of another group of patients who administered the same drug. For example, if the object of comparison is the patient's reactivity, an increase in drug reactivity based on a certain point in the series of administering a specific drug is considered an improvement of drug reactivity based on that point in time. If the object of comparison is the average of another patient group administered with the same drug, a higher drug reactivity of a specific patient, as compared to the average value of drug reactivity of a group of patients of the same cancer type that can be confirmed through a known database, is considered that the patient's drug reactivity is high.

As used herein, the term "cancer" generally refers to a physiological condition in mammals characterized by uncontrolled cell proliferation. Examples of cancer comprise colon cancer, breast cancer, glioma, thyroid cancer, lung cancer, liver cancer, pancreatic cancer, head and neck cancer, stomach cancer, urothelial cancer, kidney cancer, prostate cancer, testicular cancer, cervical cancer, ovarian cancer, endometrial cancer, melanoma, fallopian tube cancer, uterine cancer, blood cancer, bone cancer, skin cancer, brain cancer, vaginal cancer, endocrine cancer, parathyroid cancer, ureteral cancer, urethral cancer, bronchial cancer, bladder cancer, bone marrow cancer, acute myeloid leukemia, acute lymphocytic or lymphoblastic leukemia, acute or chronic lymphocytic leukemia, acute non-lymphocytic leukemia, brain tumor, cervical cancer, chronic myeloid leukemia, intestinal cancer, T-zone lymphoma, esophageal cancer, gallbladder cancer, Ewing's sarcoma, tongue cancer, Hopkins lymphoma, Kaposi's sarcoma, mesothelioma, multiple myeloma, neuroblastoma, non-Hopkin lymphoma, osteosarcoma, neuroblastoma, mammary cancer, cervical cancer, penile cancer, retinoblastoma, skin cancer, and uterine cancer, and, more preferably, acute myeloid leukemia, acute lymphoblastic or lymphoblastic leukemia, acute or chronic lymphocytic leukemia, acute non-lymphocytic leukemia, acute myeloid leukemia, or chronic myeloid leukemia, but the present invention is not limited thereto.

In the present invention, the type of the anticancer agent is not particularly limited, but may comprise, for example, DNA methyltransferase (DNMT) inhibitors (e.g. decitabine, azacytidine and RG-108), platinum-based antineoplastic agents (e.g., cisplatin, carboplatin, dicyclo Includes dicycloplatin, nedaplatin, oxaliplatin, picoplatin, and satraplatin), uucleoside analogs and antimetabolites (e.g., cytarabine, fludarabine, gemcitabine and 5FU), DNA intercalators (e.g., danorubicin, doxorubicin, epirubicin, idarubicin and camptothecin), alkylating neoplastic agents (e.g., cyclophosphamide, melphalan, bendamustine, carmustine, lomustine and ifosfamide), topoisomerase inhibitors (e.g., etoposide and topotecan), PARP inhibitors (e.g., olaparib, niraparib and rucaparib), microtubules substances that interfere with microtubule dynamics (e.g., combrestatin, eribulin, docetaxel, taxane, vinoblastine and vincristine), substances that block the interaction between p53 and MDM2 or MDM4 (e.g., nutlin, idasanutlin, HDM-201, DS3032b, AMG-232 and ALRN-6924), kinase inhibitors (e.g., BRAF inhibitors vemurafenib and dabrafenib), PI3K and/or mTOR inhibitors (e.g., LY294002, including dactolisib, rapamycin and rapamycin analogues such as temsirolimus, everolimus and ridaforolimus}, MRP1 inhibitors (e.g., indomethacin, meloxicam, sulindac sulfide, GSK1904529A, MK571 and verapamil), hypomethylation agents (e.g., azacitidine and decitabine), histone deacetylase inhibitors (e.g., circuin, hydroxamates including vorinostat, belinostat, dacinostat, panobinostat, valproic acid, benzamides including entinostat and mosetinostat), proteasome inhibitors (e.g., bortezomib, ritonavir and carfilzomib), antivascular or antiangiogenic agents (e.g., 2aG4 and bevacizumab), tyrosine kinase inhibitors (e.g., lapatinib), EGFR inhibitors (e.g., gefitinib), CDK inhibitors, PLK inhibitors, MEK inhibitors (e.g., pimasertib), immune checkpoint inhibitors (e.g., antibodies to PD-1 (e.g., nivolumab and pembrolizumab), PD-L1 (e.g., avelumab and atezolizumab), PDL2, CTLA-4 (e.g., ipilimumab and tremelimumab), GITR, IL-40, CD-40, LAG3/CD-223 (e.g., BMS-986016 and REGN3767), and OX-40 (e.g., pogalizumab and PF-04518600)), antibody binding protein tyrosine kinase receptors, NFE2L2 inhibitors (e.g., ML385, brusatol, trigonelline, luteolin, ascorbic acid and ATRA), autologous T cells that are genetically engineered to express chimeric antigen receptors (CARs) that recognize extracellular cancer targets (e.g., CD19, PSMA and mesothelin), glucocorticoid receptor agonists (e.g., dexamethasone), buthionine sulfoximine, folic acid, metformin, sorafenib, sulfasalazine, and bleo bleomycin, erlotinib, tunicamycin, wortmannin, pidilizumab, durvalumab, GSK3174998, tavolixizumab, deazaneplanocin A, or piperlongumine, and, preferably, DNA methyltransferase (DNMT) inhibitors (e.g., decitabine, azacytidine and RG-108) or DNMT inhibitors in combination with other anticancer drugs.

In the present invention, the subject refers to a vertebrate or invertebrate animal. In some embodiments, the subject is a vertebrate, such as a mammal, preferably a human being. In some embodiments, the subject is a domestic animal or laboratory animal, comprising, but not limited to, household pets such as dogs, cats, pigs, rabbits, rats, mice, gerbils, hamsters, guinea pigs and ferrets. In some embodiments, the subject is a livestock animal. Non-limiting examples of domestic animals may comprise alpacas, bison, camels, cows, deer, pigs, horses, llamas, mules, donkeys, sheep, goats, rabbits, reindeer, and yaks.

The biological sample comprises cancer tissue, cancer tissue fragments, cancer cells, blood, plasma, blood cells, saliva, tear fluid, stool, and urine separated from the subject, but the present invention is not limited thereto. Alternatively, the biological sample may be separated from cancer tissue, cancer tissue fragments, cancer cells, blood, plasma, blood cells, saliva, tear fluid, feces and urine. In addition, the DNA may be DNA separated from tissues, cells, etc., and it may be cell free DNA (cfDNA) floating in blood, plasma, body fluids, etc., or circulating tumor DNA (ctDNA) shed from tumor cells.

Methods for measuring the methylation status of CpG sites in genomic DNA as long as they are known in the art may be used without limitation and, for example, may comprise Ms-SNuPE, methylated DNA immunoprecipitation (MeDip), bisulfige sequencing, bisulfite treatment followed by next-generation sequencing, microarray, genome-wide microarray, PCR, methylation-specific PCR, real time methylation specific PCR, MethyLight PCR, MethyLight digital PCR, EpiTYPER, PCR using methylated DNA-specific binding protein, quantitative PCR, DNA chip, molecular beacon, next-generation sequencing panel (NGS panel) and methylation-sensitive southern blotting, and, preferably, bisulfige sequencing, bisulfite treatment followed by next-generation sequencing, microarray or genome-wide microarray.

In the present invention, the bisulfite sequencing may comprise whole genome bisulfite sequencing (WGBS) and reduced representation bisulfite sequencing (RRBS).

In one embodiment, CpG methylation in the entire genomic DNA or in specific regions thereof may be detected bisulfite treatment and, optionally, amplification of the methylation site, followed by pyrosequencing. Pyrosequencing technology is a real-time synthesis-specific sequencing method, which is based on indirect bioluminescence analysis of PPi released from each deoxynucleotide (dNTP) upon DNA chain elongation. This method presents a dNTP and DNA template-primer complex in the presence of exonuclease-deficient Klenow DNA polymerase. Four (4) nucleotides are sequentially added to the reaction mixture in a predetermined order. Since nucleotides are complementary to the template base, their incorporation reads to release of PPi. PPi and other reagents are used as substrates in luciferase reactions to produce visible light detected by a luminometer or charge-coupled device. The generated light is proportional to the number of nucleotides added to the DNA primer, and becomes a peak indicating the number and type of nucleotides present in the form of a pyrogram.

In another aspect, the methylation status of CpGs may be detected in the analysis of methylation using next-generation sequencing (NGS). For example, DNA methylation may be detected by massively parallel sequencing via bisulfite transitions, such as whole genome bisulfite sequencing (WGBS) or reduced representation bisulfite sequencint (RRBS). Optionally, DNA methylation may be detected by microarrays such as a genome-wide microarray. A microarray and massively parallel sequencing enable the investigation of cytosine methylation at a genome-wide scale.

One of the most comprehensive and efficient methods for detecting DNA methylation may be whole genome bisulfite sequencing (WGBS). Specifically, in order to detect CpG methylation, the DNA to be analyzed is first converted such that unmethylated cytosine is converted to uracil. In one embodiment, chemical reagents may be used to selectively modify CpG dinucleotide motifs in methylated or demethylated form. Suitable chemical reagents may comprise hydrazine and bisulfite. Preferably, the isolated DNA may be treated with bisulfite, which converts unmethylated cytosine to uracil while retaining methylated cytosine. Cytosine reacts with bisulfite ions to form a sulfonated cytosine reaction intermediate that is susceptible to deamination, thereby yielding sulfonated uracil. The sulfonated group may be removed under alkaline conditions to form uracil. Nucleotide conversions change the sequence of DNA. Uracil is recognized as thymine by DNA polymerase. Therefore, the result after PCR or sequencing comprises cytosine only at the position where 5-methyl cytosine occurs in the starting template DNA. This allows distinguishing between unmethylated and methylated cytosines.

In one aspect of the invention, the methylation disorder of the adjacent CpG sites may be assessed according to a method comprising the following steps: (i) calculating (A) the number of sequencing reads in which CpG sites contained in sequencing read mapped to each CpG site are all methylated or all demethylated, and (B) the number of remaining sequencing reads, in one or more regions in genomic DNA; (ii) calculating the value (B/(A+B)) of methylation disorder of each CpG site; and (iii) calculating the average of the values of methylation disorder calculated from all CpG sites contained in one or more regions in the genomic DNA.

The sequencing reads are mapped to a reference sequence to determine to which region of genomic DNA the sequencing read corresponds in order to calculate the methylation disorder of adjacent CpG sites. From these results, the methylation disorder is calculated as follows.

For each CpG site in the reference sequence, (A) the number of sequencing reads in which 2 to 20 CpG sites adjacent to each other among the CpG sites contained in the sequencing read corresponding to that site (i.e. mapped to that site) are all methylated or all demethylated, and (B) the number of remaining sequencing reads are calculated. Next, the methylation disorder is calculated as B/(A+B).

Such calculation is specified as follows.

**(Calculation of methylation disorder at a specific CpG site-level)** for example, it is assumed that there are a total of 100 sequencing reads mapped to a specific CpG site (x) in genomic DNA, the number of reads in which the CpG sites containing x in the read are all methylated is 35, and the number of reads in which the CpG sites containing x in the reads are all demethylated is 5. Then, the number of reads in which both methylated and demethylated CpG sites exist simultaneously is 100-35-5=60. Thus, the methylation disorder calculated in step (ii) is calculated as 60/100 = 0.6.

**(Calculation of methylation disorder at a level of genome-wide or specific regions within the genome)** Based on the above-mentioned methylation disorder at a level of genome-wide or specific regions within the genomic DNA is performed. In this case, the methylation disorder at a level of genome-wide or specific regions within the genome is defined as the average of the disorder values of specific CpG site-level methylation at all CpG sites contained in the entire genomic DNA or in one or more regions of genomic DNA for which the value is to be calculated.

The CpG site contained in the calculation of the average value may preferably be CpG sites mapped to 11 or more reads simultaneously comprising at least 4 CpG sites.

In another aspect of the present invention, the methylation disorder of adjacent CpG sites may be calculated by epipolymorphism, methylation entropy, methylation haplotype load (MHL), fraction of discordant read pair (FDRP) or quantitative FDRP.

The concept underlying the calculation of epipolymorphism and methylation entropy is an epiallele. In the calculations below, an epiallele is defined as a combination or pattern of methylation status formed by four (4) consecutive CpG sites. Since each CpG site has two states: methylation (1) or demethylation (0), the total number of the patterns of methylation status created by the four (4) CpG sites constituting one epiallele is 2*2*2*2=16 (from the pattern (0, 0, 0, 0) where CpG sites are all demethylated, to the pattern (1, 1, 1, 1) where CpG sites are all methylated). Therefore, there are a total of 16 types of epialleles that may occur in specific genomic regions corresponding to the four (4) CpG sites.

Intuitively, as the methylation disorder increases, the types of epialleles may also become more diverse. This is because epigenetic alleles that are all methylated or all demethylated will predominantly appear in regions with a low degree of disorder, and many other epigenetic alleles will also appear in regions with a high degree of disorder. Based on this concept, epipolymorphism and methylation entropy are to calculate the methylation disorder by calculating the diversity of epigenetic alleles as follows.

Epipolymorphism is a method of calculating the methylation disorder by borrowing the method of calculating the Gini-Simpson index, which is used to measure species diversity in ecology. First, the sequencing reads comprising all four (4) CpG sites for which epipolymorphism is to be calculated are traversed, and the frequency of each of sixteen (16) possible epialleles created by the four (4) CpG sites is measured. When the relative frequency value of sixteen (16) epialleles is p_i (i=1, 2, ... 16), epipolymorphism is calculated as follows.

$$1 - \sum_{i=1}^{16} p_i^2$$

This value becomes smallest at 0 when only one pattern among the 16 epiallele patterns appears, and becomes maximum when the relative frequencies of all 16 patterns are the same at 1/16. Therefore, it may be a measure of the diversity of patterns, which means that it may be a measure of the methylation disorder.

Similar to epipolymorphism, methylation entropy is a method of measuring the relative diversity of epigenetic alleles, but differs in that it uses Shannon entropy instead of the Gini-Simpson index. The methylation entropy value is calculated as follows.

$$-\frac{1}{4}\sum_{i=1}^{16} p_i \log_2 p_i$$

This value also becomes smallest at 0 when only one of the 16 epigenetic allele types appears, and becomes maximum when the relative frequencies of all 16 patterns are the same at 1/16.

Methylation haplotype load (MHL) aims to detect regions where the correlation of methylation status is locally very high. The methylation haploid load is calculated by finding, based on the methylation status of the CpG site contained in each sequencing read, fully-methylated substrings contained therein, and may be expressed mathematically as follows.

$$\mathrm{MHL} = \frac{\sum_{i=1}^{l} w_i \times P(MH_i)}{\sum_{i=1}^{l} w_i}$$

Here, P(MH_i) represents the fraction of substrings in which CpG sites are all methylated among substrings with i CpG sites, and w_i is a weight given to the length of the substrings. Usually, w_i = i, that is, the length of the substrings *per se* is used as a weight, but w_i = i^2 may be used to assign a greater weight to a long methylated haploid.

For example, it is assumed that there is a sequencing read and a total of 5 CpG sites therein, and each methylation status is 0, 1, 1, 1, 0 (0=demethylation, 1=methylation). Next, substrings of any length are considered. First, the substrings of length 1 are 0, 1, 1, 1, and 0, respectively. Among them, there are three fully-methylated substrings: 1, 1, and 1, and the fraction of fully-methylated substrings is 3/5 = 0.6. Next, the substrings of length 2 are 01, 11, 11, and 10. Among them, there are two fully-methylated substrings, 11 and 11, and the fraction of fully-methylated substrings is 2/4 = 0.5. This value is multiplied by a weight of 2. Likewise, the substrings of length 3 are 011, 111, and 110. Among them, there is a fully-methylated substring: 111, and the fraction of fully-methylated substrings is 1/3. This value is multiplied by a weight of 3. There are two substrings of length 4, 0111 and 1110, but there are no fully-methylated substrings among them. As a result, the methylation haploid load of this sequencing read is (3/5 * 1 + 2/4 * 2 + 1/3 * 3) / (1 + 2 + 3 + 4) = 0.26.

Fraction of discordant read pair (FDRP) is a method of mearusing the disorder of DNA methylation at the level of a single CpG site. First, when calculating the FDRP value of a specific CpG site x, all sets of sequencing reads containing x are considered, and such sets are called as S. If all sequencing read pairs contained in S, i.e., s_i and s_j, are different from each other in terms of the methylation status of at least one CpG site commonly contained in s_i and s_j, s_i and sj are defined as a discordant read pair. FDRP is obtained by dividing the number of discordant read pairs by the number of all sequencing read pairs in S.

For example, it is assumed that the two sequencing reads s_i and s_j contained in S have four CpG sites x, y, z, and w in common (since an FDRP for x is being calculated, the common CpG sites in any two read pairs as selected must contain x). If the methylation status of x, y, z, and w on s_i is called 1, 1, 1, 1 (0=demethylation, 1=methylation), and the methylation status of x, y, z, and w on s_j is 1, 1, 0, 1, the methylation status of the CpG site z is different between the two sequencing read pairs, which become a discordant read pair. The FDRP value is a measure of the fraction of such discordant read pairs among the read pairs in S.

Meanwhile, quantitative FDRP (qFDRP) is a method of softly calculating the criteria for discordance based on the hamming distance, rather than dichotomously judging same. In other words, qFDRP is an expected value of the fraction of sites having a different methylation status from each other among the CpG sites commonly contained in the sequencing read pairs s_i and sJ contained in S, which is a set S of all sequencing reads containing a specific CpG site x.

For example, it is assumed that S, the set of sequencing reads containing CpG site x, has a total of three (3) sequencing reads (i.e., S=(s_1, s_2, s_3)), s_1 and s_2 contain four (4) CpG sites in common. If one CpG site among them is different from each other, the Hamming distance of the two reads is 1, and the fraction of sites with different methylation status is 1/4 = 0.25. If such value is calculated for pair (s_2, s_3) and pair (s_3, s_1) in the same way, and 0.5 and 0.2, respectively, are obtained, the total qFDRP value is (0.25 + 0.5 + 0.2) / 3 = 0.316.

In another aspect of the invention, the methylation disorder of adjacent CpG sites may be assessed according to a method comprising: (i) calculating (A) the number of sequencing reads in which two CpG sites located adjacent to each other by one or more distances selected from the group consisting of 2 to 100 bp are all methylated or all demethylated, and (B) the number of remaining sequencing reads, in one or more regions in genomic DNA; and (ii) calculating the value (B/(A+B)).of methylation disorder of the CpG sites located adjacent to each other by each distance

In general, when analyzing the sequence of genomic DNA, the sequencing read may have a length of about 100 base pairs (bp), and, in step (i), among a plurality of CpG sites contained in the sequencing read corresponding to one or more regions in the genomic DNA (e.g., regions selected from a group consisting of an entire genomic DNA, a bivalent domain, a promoter, an enhancer, an exon, an intron, a 5'-untranslated region (UTR), a 3'-UTR, a gene body, a stem cell-related region, a CpG island, a CpG shore, a long terminal repeat (LTR), a long interspersed nuclear element (LINE), a short interspersed nuclear element (SINE), a CpG shelf, a methylation canyon, and an intergenic region), the methylation status of two CpG sites having a genetic distance of 2 bp, 3 bp, 4 bp, 5 bp, 6 bp, 7 bp, 8 bp, 9 bp, 10 bp, 11 bp, 12 bp, 13 bp, 14 bp, 15 bp, 16 bp, 17 bp, 18 bp, 19 bp, 20 bp, 21 bp, 22 bp, 23 bp, 24 bp, 25 bp, 26 bp, 27 bp, 28 bp, 29 bp, 30 bp, 31 bp, 32 bp, 33 bp, 34 bp, 35 bp, 36 bp, 37 bp, 38 bp, 39 bp, 40 bp, 41 bp, 42 bp, 43 bp, 44 bp, 45 bp, 46 bp, 47 bp, 48 bp, 49 bp, 50 bp, 51 bp, 52 bp, 53 bp, 54 bp, 55 bp, 56 bp, 57 bp, 58 bp, 59 bp, 60 bp, 61 bp, 62 bp, 63 bp, 64 bp, 65 bp, 66 bp, 67 bp, 68 bp, 69 bp, 70 bp, 71 bp, 72 bp, 73 bp, 74 bp, 75 bp, 76 bp, 77 bp, 78 bp, 79 bp, 80 bp, 81 bp, 82 bp, 83 bp, 84 bp, 85 bp, 86 bp, 87 bp, 88 bp, 89 bp, 90 bp, 91 bp, 92 bp, 93 bp, 94 bp, 95 bp, 96 bp, 97 bp, 98 bp, 99 bp, or 100 bp may be assessed.

In this way, the methylation status of two adjacent CpG sites having a genetic distance selected from the group consisting of 2 to 100 bp may be compared and assessed among CpG sites contained in the entire genomic DNA or in a specific region within the genomic DNA to calculate (A) the number of sequencing reads that are all methylated or all demethylated, and (B) the number of remaining sequencing reads, for every two adjacent CpG sites with the genetic distance in the genomic DNA.

Thereafter, in step (ii), the methylation disorder of two adjacent CpG sites having a genetic distance selected from the group consisting of 2 to 100 bp may be calculated according to the following equation:

$$[\text{Methylation disorder of adjacent CpG sites} = B/(A+B)]$$

If the methylation disorder of CpG sites adjacent to the genetic distance among the CpG sites contained in the entire genomic DNA or in a specific region in the genomic DNA is calculated according to the equation, the average value may be taken as the value of [methylation disorder of adjacent CpG sites] for the genetic distance and the [methylation disorder of adjacent CpG sites] of the subject.

In one aspect of the present invention, in steps (i) and (ii), the value of [methylation disorder of adjacent CpG sites] may be calculated for each two adjacent CpG sites having a genetic distance of 2 or more, and then their average value may be calculated. For example, the value of [methylation disorder of adjacent CpG sites] in the entire genomic DNA or in a specific region within the genomic DNA may be calculated for two adjacent CpG sites with a genetic distance of 2 bp, and, independently thereof, the value of [methylation disorder of adjacent CpG sites] in the entire genomic DNA or in a specific region within the genomic DNA may be calculated for two adjacent CpG sites with a genetic distance of 3 bp, and then the average value may be taken as the [methylation disorder of adjacent CpG sites] of the subject. In this way, among the CpG sites contained in the entire genomic DNA or in a specific region in the genomic DNA, the value of [methylation disorder of adjacent CpG sites] of each of two adjacent CpG sites with at least one genetic distance selected from the group consisting of 2 to 100 bp may be calculated, and then the average value may be taken as the [methylation disorder of adjacent CpG sites] of the subject.

Preferably, in step (i), the methylation status of two adjacent CpG sites with one or more genetic distances selected from the group consisting of 2 to 50 bp, more preferably 2 to 30 bp, and most preferably 2 to 25 bp, among the CpG sites contained in the entire genomic DNA or in a specific region within the genomic DNA may be compared and assessed to calculate the value of [methylation disorder of adjacent CpG sites] of the subject.

According to one embodiment of the present invention, subjects with higher values of [methylation disorder of adjacent CpG sites] of the CpG sites contained in the entire genomic DNA or in a specific region within the genomic DNA calculated by the above-describe method showed higher reactivity to anticancer drugs.

Therefore, the present invention may be characterized in that subjects with a higher degree of methylation disorder of adjacent CpG sites are expected to have higher reactivity to anticancer drugs.

In particular, it was confirmed that prediction of reactivity to anticancer drugs based on [methylation disorder of adjacent CpG sites] was particularly useful for anticancer drugs whose reactivity is hardly predicted through methylation level analysis.

**ADVANTAGEOUS EFFECTS OF INVENTION**

The method of the present invention can accurately predict reactivity to anticancer drugs by using the methylation disorderness of CpG sites in genomic DNA, and thus can be very useful in the selection of drugs for cancer patient treatment.

## BRIEF DESCRIPTION OF DRAWINGS

FIG. 1 is a schematic diagram showing a method for calculating an LPMD value, as an example of [methylation disorder of adjacent CpG sites], in the embodiments of the present invention.

FIG. 2 is a diagram showing the correlation between [methylation disorder of adjacent CpG sites (LPMD)] in the entire DNA of an acute myeloid leukemia cancer cell line and area under the drug response curve for each displayed anticancer drug (AUDRC).

FIGS. 3 to 11 are diagrams showing correlation between [methylation disorder of adjacent CpG sites] in a specific region of DNA of acute myeloid leukemia cancer cell line and area under the drug response curve for each displayed anticancer drug (AUDRC).

FIG. 12 is a diagram showing correlation between [methylation disorder of adjacent CpG sites (LPMD)] values of 454 genomic regions confirmed to well represent the DNA methylation disorder of each genome, comprising bivalent domains in blood samples from a total of 22 patients diagnosed with acute myeloid leukemia who were prescribed demethylating anticancer drugs (Decitabine or Azacitinde), and patient's drug reactivity.

## MODE FOR INVENTION

Hereinafter, the present invention will be described in detail by the following embodiments. However, the following embodiments are only for illustrating the present invention, and the present invention is not limited thereto.

### Example 1: *In vitro* analysis

The following experiment was performed to confirm the correlation between the [methylation disorder of adjacent CpG sites] value and reactivity to anticancer drugs.

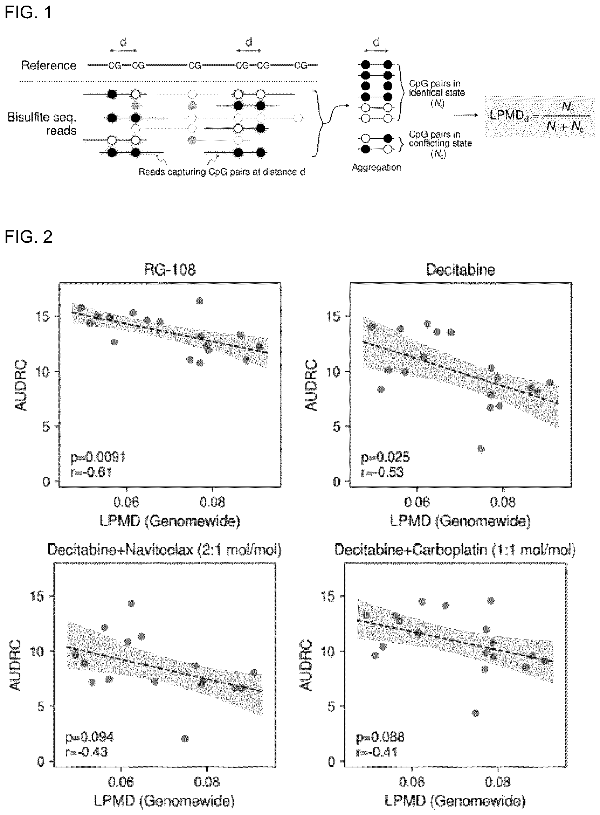

First, RRBS sequencing results for an acute myeloid leukemia cancer cell line listed in the Cancer Cell Line Encyclopedia (CCLE) were used. The methylation status of two adjacent CpGs located at a genetic distance of 2bp was confirmed in the sequencing reads mapped to the reference sequence, and (A) the number of sequencing reads in which the two CpGs were all methylated or all demethylated and (B) the number of remaining sequencing reads were calculated. B/(A+B) was taken as the [methylation disorder of adjacent CpG sites by a genetic distance of 2bp] (FIG. 1).

In the same way, the value of [methylation disorder of adjacent CpG site] was calculated for each of two adjacent CpGs with a genetic distance of 3bp, 4bp, 5bp, 6bp, 7bp, 8bp, 9bp, 10bp, 11 bp, 12bp, 13bp, 14bp, 15bp, 16bp, 17bp, 18bp, 19bp, 20bp, 21 bp, 22bp, 23bp or 24bp in the sequencing reads mapped to the reference sequence, and the value of the methylation disorder of each of adjacent CpG sites with a genetic distance ranging from 2 bp to 24 bp were averaged. The average value is taken as the value of [methylation disorder of adjacent CpG sites] of the cancer cell line, and is called an LPMD value.

Meanwhile, the value of the reactivity to anticancer drugs of the cancer cell line was obtained and prepared in the form of [area under the drug response curve] (AUDRC) from the Cancer Therapeutics Response Portal (CTRP). The present embodiment presents results on drug reactivity to the DNMT inhibitor decitabine (administration of decitabine alone, combined administration of decitabine and navitoclax, or combined administration of decitabine and carboplatin), and RG-108.

The results are shown in FIG. 2.

As shown in FIG. 2, it was confirmed that the value of [methylation disorder of adjacent CpG sites] in the entire DNA of acute myeloid leukemia cancer cell lines and the AUDRC value for each drug showed a significant negative correlation in the four (4) drug-treated groups. At this time, since a decrease in the AUDRC value indicates good drug reactivity of the cancer cell line, it is confirmed that the value of [methylation disorder of adjacent CpG sites] has predictive power for reactivity to anticancer drugs.

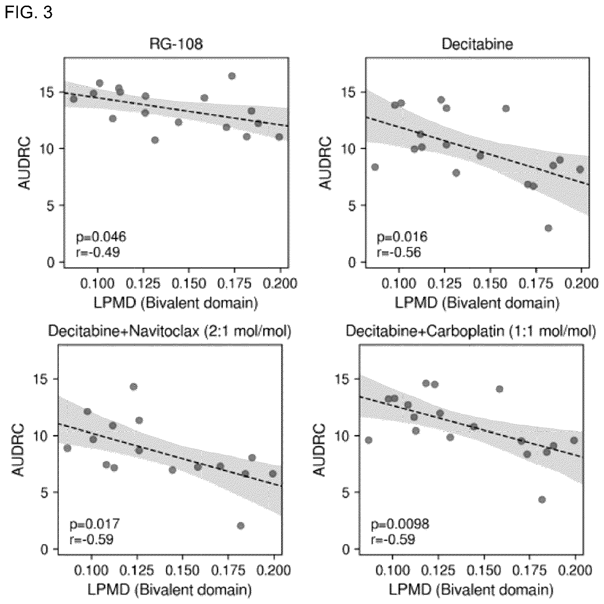

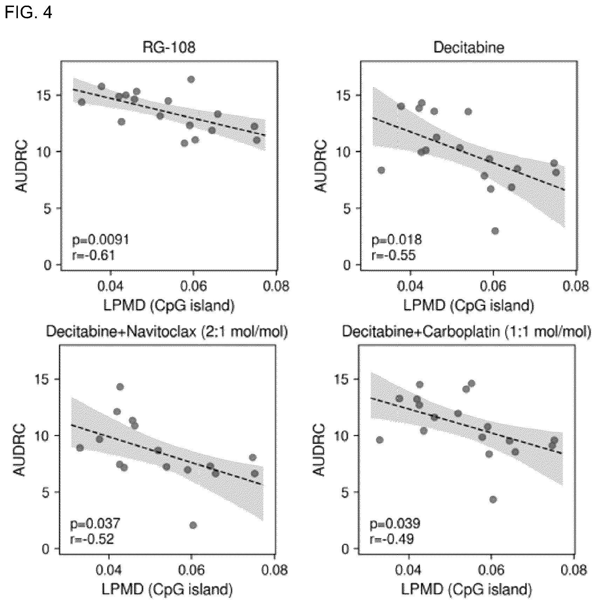

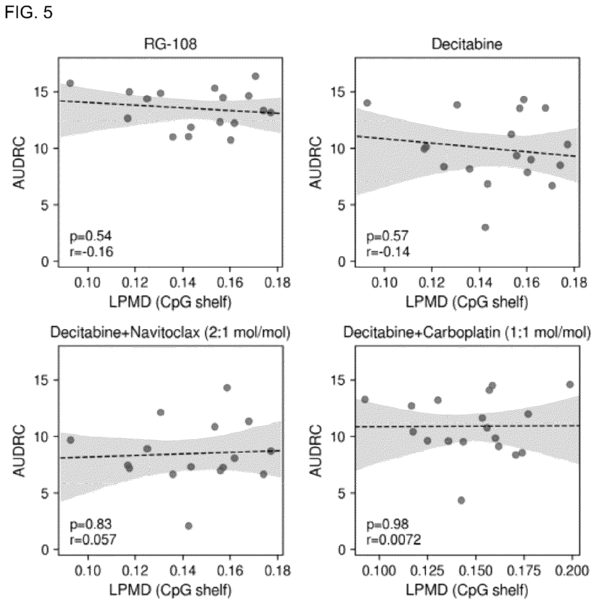

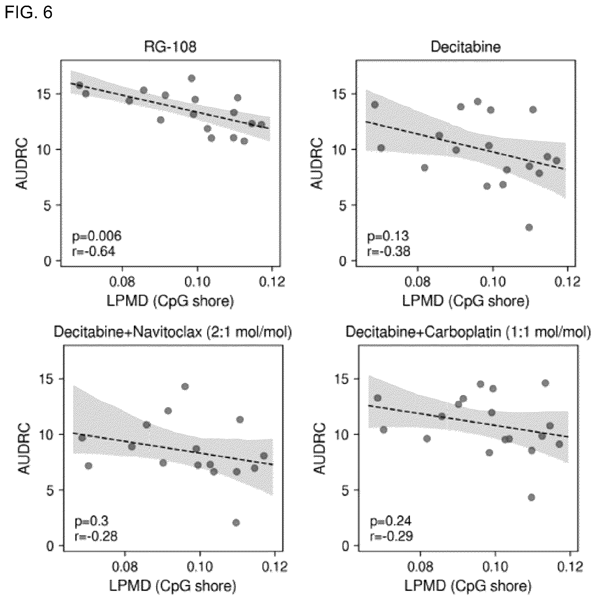

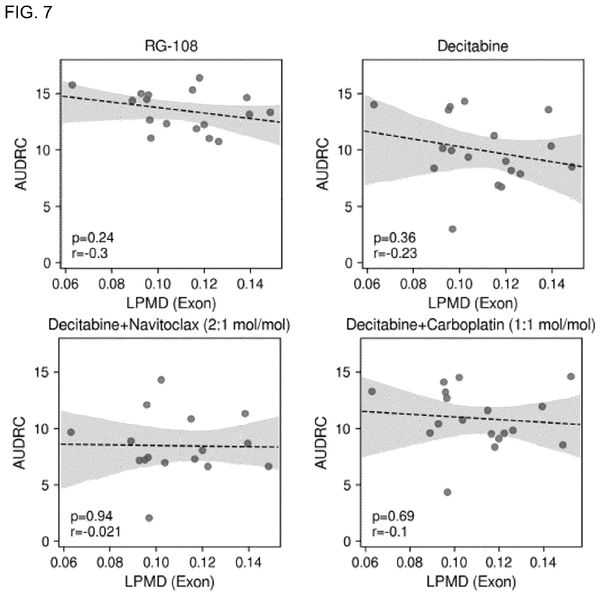

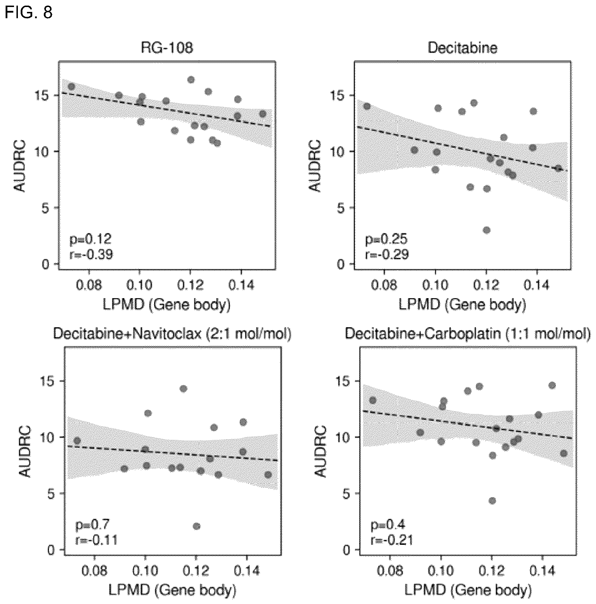

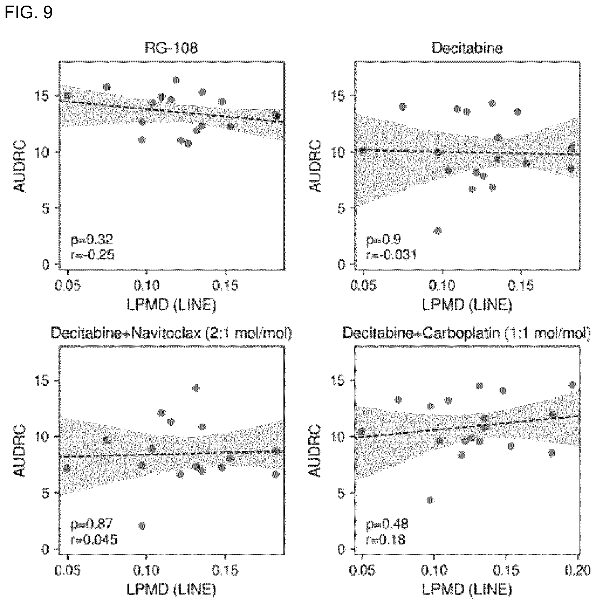

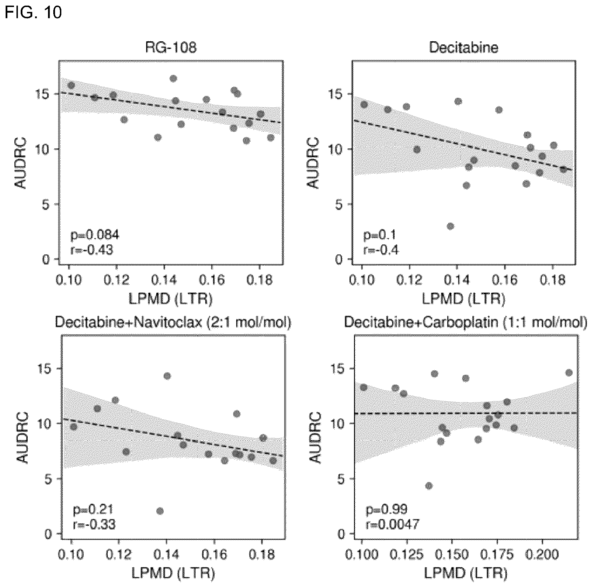

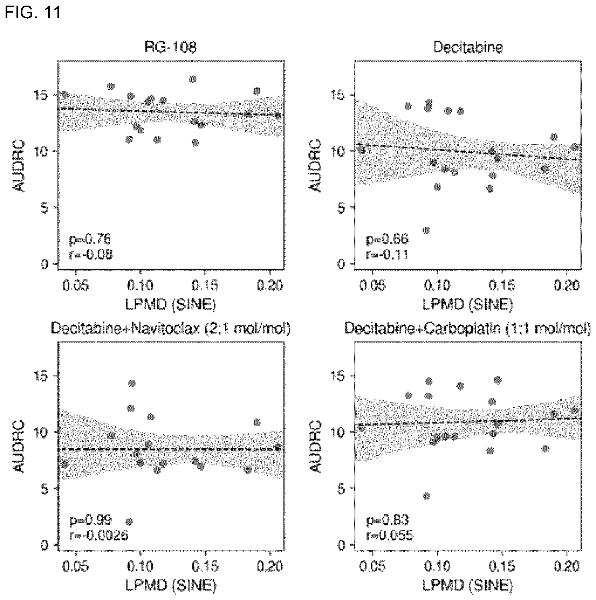

In addition, as shown in FIGS. 3 to 11, the value of [methylation disorder of adjacent CpG sites] in specific regions within DNA also showed a significant negative correlation with the AUDRC value for each drug. Thus, it is confirmed that the predictive power of the [methylation disorder of adjacent CpG sites] for drug reactivity may be exerted both in the entire genomic DNA and in specific regions within the genomic DNA.

### Example 2: Analysis of clinical data

Targeted bisulfite sequencing data was produced for blood samples of a total of 22 patients who were prescribed demethylating anticancer drugs (Decitabine or Azacitinde) among patients diagnosed with acute myeloid leukemia at Seoul National University Hospital. The regions where sequencing was performed were 454 genomic regions that were identified as being well representative of the disorder of DNA methylation of each genome, mainly comprising bivalent domains. The patients were divided into two groups of those who survived for more than 9 months or achieved complete remission (good response), and those who did not (poor response), according to reactivity to a demethylating agent.

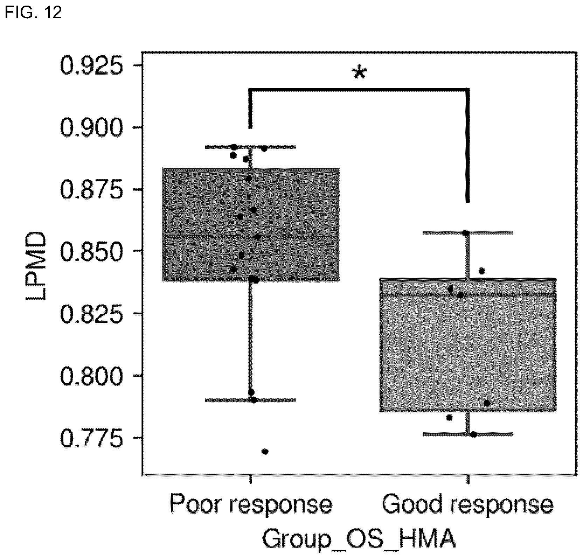

The results are shown in FIG. 12.

As shown in FIG. 12, it was confirmed that the LPMD value, which is an example of the value of methylation disorder of adjacent CpG sites, was significantly reduced in the patient group showing good response (Mann-Whitney U test p=0.017). In these results, the LPMD was calculated using CpG pairs with distances of from 2 to 16, which shows that the level of methylation disorder is associated with reactivity to anticancer drugs not only in cell lines but also in actual patient blood samples, thereby suggesting the possibility of clinical application of the present invention.

## INDUSTRIAL APPLICABILITY

The method of the present invention can accurately predict reactivity to anticancer drugs by using the methylation disorderness of CpG sites in genomic DNA, and thus can be very useful in the selection of drugs for cancer patient treatment. Therefore, the present invention has a high industrial applicability.

## Claims

1. A method for assessing the methylation status of adjacent CpG sites in genomic DNA in order to provide information necessary to predict cancer patient's reactivity to anticancer drugs, the method comprising:

   (a) isolating genomic DNA from a biological sample isolated from a subject;
   (b) measuring the methylation status of CpG sites in the isolated genomic DNA; and
   (c) assessing the methylation disorder of adjacent CpG sites in one or more regions in the genomic DNA.

2. The method of claim 1, wherein the cancer is selected from the group consisting of colon cancer, breast cancer, glioma, thyroid cancer, lung cancer, liver cancer, pancreatic cancer, head and neck cancer, stomach cancer, urothelial cancer, kidney cancer, prostate cancer, testicular cancer, cervical cancer, ovarian cancer, endometrial cancer, melanoma, fallopian tube cancer, uterine cancer, blood cancer, bone cancer, skin cancer, brain cancer, vaginal cancer, endocrine cancer, parathyroid cancer, ureteral cancer, urethral cancer, bronchial cancer, bladder cancer, bone marrow cancer, acute myeloid leukemia, acute lymphocytic or lymphoblastic leukemia, acute or chronic lymphocytic leukemia, acute non-lymphocytic leukemia, brain tumor, cervical cancer, chronic myeloid leukemia, intestinal cancer, T-zone lymphoma, esophageal cancer, gallbladder cancer, Ewing's sarcoma, tongue cancer, Hopkins lymphoma, Kaposi's sarcoma, mesothelioma, multiple myeloma, neuroblastoma, non-Hopkin lymphoma, osteosarcoma, neuroblastoma, mammary cancer, cervical cancer, penile cancer, retinoblastoma, skin cancer, and uterine cancer.

3. The method of claim 1, wherein the anticancer drug comprises a DNA methyltransferase (DNMT) inhibitor.

4. The method of claim 3, wherein the DNMT inhibitor is one or more selected from the group consisting of decitabine, azacytidine, RG-108 and a salt thereof.

5. The method of claim 1, wherein the biological sample is selected from the group consisting of cancer tissue, cancer tissue fragments, cancer cells, blood, plasma, blood cells, saliva, tear fluid, feces and urine.

6. The method of claim 1, wherein the step of measuring the methylation status is conducted by a method selected from the group consisting of Ms-SNuPE, methylated DNA immunoprecipitation (MeDip), bisulfige sequencing, bisulfite treatment followed by next-generation sequencing, microarray, genome-wide microarray, PCR, methylation-specific PCR, real time methylation specific PCR, MethyLight PCR, MethyLight digital PCR, EpiTYPER, PCR using methylated DNA-specific binding protein, quantitative PCR, DNA chip, molecular beacon, next-generation sequencing panel (NGS panel) and methylation-sensitive southern blotting.

7. The method of claim 6, wherein the bisulfite sequencing is whole genome bisulfite sequencing (WGBS) or reduced representation bisulfite sequencing (RRBS).

8. The method of claim 1, wherein the one or more regions in the genomic DNA is selected from a group consisting of an entire genomic DNA, a bivalent domain, a promoter, an enhancer, an exon, an intron, a 5'-untranslated region (UTR), a 3'-UTR, a gene body, a stem cell-related region, a CpG island, a CpG shore, a long terminal repeat (LTR), a long interspersed nuclear element (LINE), a short interspersed nuclear element (SINE), a CpG shelf, a methylation canyon and an intergenic region.

9. The method of claim 1, wherein the methylation disorder of adjacent CpG sites is assessed according to a method comprising the following steps of:

(i) calculating (A) the number of sequencing reads in which CpG sites contained in sequencing reads mapped to each CpG site are all methylated or all demethylated, and (B) the number of remaining sequencing reads, in one or more regions in genomic DNA;
(ii) calculating the value (B/(A+B)) of methylation disorder of each CpG site; and
(iii) calculating the average of the values of methylation disorder calculated from all CpG sites contained in one or more regions in the genomic DNA.

10. The method of claim 1, wherein the methylation disorder of adjacent CpG sites is assessed according to a method comprising the following steps of:

(i) calculating (A) the number of sequencing reads in which two CpG sites located adjacent to each other by one or more distances selected from the group consisting of 2 to 100 bp are all methylated or all demethylated, and (B) the number of remaining sequencing reads, in one or more regions in genomic DNA; and
(ii) calculating the value (B/(A+B)) of methylation disorder of the CpG sites located adjacent to each other by each distance.

11. The method of claim 10, wherein, after step (ii), the method further comprises calculating the average of the values of methylation disorder of adjacent CpG sites located adjacent to each other by each distance.

12. The method of claim 1, wherein the methylation disorder of adjacent CpG sites is assessed by epipolymorphism, methylation entropy, methylation haplotype load (MHL), fraction of discordant read pair (FDRP) or quantitative FDRP.

13. The method of claim 1, wherein subjects with a higher degree of methylation disorder of adjacent CpG sites are expected to have higher reactivity to anticancer drugs.

14. The method of claim 1, wherein the subject refers to a vertebrate or invertebrate animal.

## FIG. 1

## FIG. 2

FIG. 3

FIG. 4

FIG. 5

FIG. 6

FIG. 7

FIG. 8

FIG. 9

FIG. 10

FIG. 11

RG-108

Decitabine

Decitabine+Navitoclax (2:1 mol/mol)

Decitabine+Carboplatin (1:1 mol/mol)

FIG. 12

## INTERNATIONAL SEARCH REPORT

| International application No. |
|---|
| **PCT/KR2022/017269** |

| A. | CLASSIFICATION OF SUBJECT MATTER |
|---|---|
| | **C12Q 1/6886**(2018.01)i |

According to International Patent Classification (IPC) or to both national classification and IPC

| B. | FIELDS SEARCHED |
|---|---|

Minimum documentation searched (classification system followed by classification symbols)

C12Q 1/6886(2018.01); C12N 15/09(2006.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항암제 반응성 예측(anti-cancer drug response prediction), 인접 CpG 메틸화 무질 서도(contiguous CpG methylation disorderness), DNMT 저해제(DNMT inhibitor), WGBS(whole genome bisulfite sequencing), 시퀀싱 리드(sequencing read)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br><br>A | KOLDOBSKIY, Michael A. et al. A dysregulated DNA methylation landscape linked to gene expression in MLL-rearranged AML. Epigenetics. 2020, vol. 15, no. 8, pp. 841-858.<br>See abstract; pages 842-843 and 852-854; and figure 1. | 1-8,12,14<br><br>9-11,13 |
| A | LV, Wenhua et al. Exploration of drug-response mechanism by integrating genetics and epigenetics across cancers. Epigenomics. 2018, vol. 10, no. 7, pp. 993-1010 (inner pp. 1-18).<br>See entire document. | 1-14 |
| A | FEINBERG, Andrew P. et al. Epigenetic modulators, modifiers and mediators in cancer aetiology and progression. Nature Reviews Genetics. 2016, vol. 17, pp. 284-299 (inner pp. 1-16).<br>See entire document. | 1-14 |
| A | SHI, Jiejun et al. The concurrence of DNA methylation and demethylation is associated with transcription regulation. Nature Communications. 2021 (online publication date: 06 September 2021), vol. 12, article no. 5285, pp. 1-12.<br>See entire document. | 1-14 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 February 2023** | **14 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2022/017269** |

**C.      DOCUMENTS CONSIDERED TO BE RELEVANT**

| Category* | Citation of document, with indication, where appropriate, of the relevant passages | Relevant to claim No. |
|---|---|---|
| A | JP 2020-520235 A (PRESIDENT & FELLOWS OF HARVARD COLLEGE et al.) 09 July 2020 (2020-07-09)<br>    See entire document. | 1-14 |

Form PCT/ISA/210 (second sheet) (July 2022)

INTERNATIONAL SEARCH REPORT
Information on patent family members

International application No.

**PCT/KR2022/017269**

| Patent document cited in search report | | | Publication date (day/month/year) | Patent family member(s) | | | Publication date (day/month/year) |
|---|---|---|---|---|---|---|---|
| JP | 2020-520235 | A | 09 July 2020 | CA | 3063405 | A1 | 15 November 2018 |
| | | | | CN | 111417731 | A | 14 July 2020 |
| | | | | EP | 3622091 | A2 | 18 March 2020 |
| | | | | US | 2020-0109456 | A1 | 09 April 2020 |
| | | | | WO | 2018-209361 | A2 | 15 November 2018 |
| | | | | WO | 2018-209361 | A3 | 10 January 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20210151412